# EUROPÄISCHE PATENTSCHRIFT

(11) **EP 0 495 247 B1**
(45) Veröffentlichungstag und Bekanntmachung des Hinweises auf die Patenterteilung: **04.09.1996**
(21) Anmeldenummer: 91122238.8
(22) Anmeldetag: 27.12.1991
(51) Int. Cl.: A61B 3/024

(54) **Verfahren zum Perimetrieren des Gesichtsfeldes eines Auges**
Process for the perimetry of the visual field of the eye
Procédé de périmétrie du champ visuel de l'oeil

(30) Priorität: 18.01.1991 CH 140/91
(43) Veröffentlichungstag der Anmeldung: 22.07.1992
(73) Patentinhaber: Interzeag AG, CH-8952 Schlieren (CH)
(72) Erfinder: Weijland, Albert, CH-8621 Wetzikon (CH)
(74) Vertreter: Fillinger, Peter, Dr.

(56) Entgegenhaltungen:
- EP-A- 0 320 767
- EP-A- 0 363 610
- US-A- 4 012 128
- US-A- 4 083 365
- Reim, Augenheilkunde, Stuttgart 1985, Seiten 108-111

## Beschreibung

Die vorliegende Erfindung bezieht sich auf ein Verfahren zum Bestimmen des Schwellenwertes der Wahrnehmung von Licht an wählbaren Testorten des Gesichtsfeldes eines Auges gemäss dem Oberbegriff des Patentanspruchs 1 sowie auf eine Einrichtung zur Untersuchung des Gesichtsfeldes des Auges eines Patienten gemäss dem Oberbegriff des Patentanspruchs 10.

Die bekannte perimetrische Untersuchung des Auges eines Patienten, das heisst das Messen der Lichtunterschiedsempfindlichkeit der Netzhaut des Auges, basiert darauf, Testorten, die vorbestimmte Punkte eines Rastersauf einer zu einer Beobachtungsachse quer orientierten Fläche sind, nacheinander einen Stimulus bestimmter Lichtintensität darzubieten, wobei der Patient das Wahrnehmen oder Nichtwahrnehmen des Stimulus durch Betätigen eines Antwortknopfs meldet. Nach einer solchen ersten Untersuchung wird an den gleichen Testorten oder einem Teil der Testorte eine zweite Untersuchung mit einem Stimulus veränderter Lichtintensität durchgeführt, an welche sich weitere Untersuchungen mit veränderter Lichtintensität des Stimulus anschliessen, bis ein Schwellenwert der Wahrnehmung empirisch und rechnerisch ermittelt ist. Bekannte Perimetrier verfahren sind aus Reim M., Augenheilkunde (Stuttgart 1985, Seite 108 - 111) bekannt; eine Einrichtung zur Untersuchung des Gesichtsfeldes ist aus EP-A-0 363 610 bekannt.

Es ist einzusehen, dass dieses bekannte Verfahren zeitraubend ist und zudem wegen der überaus zahlreichen erforderlichen Entscheidungen den Patienten stark belastet, was die Zuverlässigkeit der Messungen beeinträchtigt.

Zur zeitlichen Abkürzung der aufeinanderfolgenden Untersuchungen und zur Erhöhung deren Genauigkeit ist es bereits bekannt, während und/oder nach einer Untersuchung mindestens die für vorbestimmte Testorte im Speicher gespeicherten Vorkenntnisse als Funktion der gemessenen, individuellen Schwellenwerte zu korrigieren und für nächst spätere Untersuchungen abzuspeichern, um bei der nächst späteren Untersuchung an den vorbestimmten Testorten zuerst einen Stimulus darzubieten, dessen Wert den korrigierten Vorkenntnissen entspricht. Aber auch diesem verbesserten Verfahren haftet der Nachteil an, dass an diesen Testorten dennoch der aktuelle Schwellenwert in einem Eingabelungsverfahren durch das Darbieten mehrerer Stimuli ermittelt werden muss.

Aufgabe der vorliegenden Erfindung ist, die Bestimmung des Schwellenwerts grundsätzlich zu verbessern und die Anzahl der insgesamt an allen Testorten erforderlichen Untersuchungsschritte namhaft zu vermindern.

Zur Lösung dieser Aufgabe weist das erfindungsgemässe Verfahren bzw. die Einrichtung der eingangs genannten Art die im kennzeichnenden Teil des Patentanspruchs 1 bzw. 10 angeführten Merkmale auf.

Da an jedem Testort ein Stimulus mit definiert zunehmendem Wert dargeboten wird, erübrigt es sich, die Testorte mehrfach mit Stimuli unterschiedlicher Werte zu beaufschlagen, so dass die Gesamtuntersuchung erheblich beschleunigt wird.

Gemäss Anspruch 2 kann der Stimulus kontinuierlich zunehmenden Wertes ein getakteter Stimulus sein.

Gemäss den Ansprüchen 3 und 4 kann der zunehmende Wert des Stimulus die Lichtintensität bzw. die Grösse des Stimulus sein.

Wie bei den bekannten Verfahren kann gemäss Anspruch 5 auch beim erfindungsgemässen Verfahren die Reaktion des Patienten auf den am gleichen Testort veränderlichen Wert des Stimulus darin bestehen, dass der Patient bei der Wahrnehmung des Stimulus einen Antwortknopf betätigt. Diese Art der Reaktion hat jedoch den Nachteil, dass die Zuverlässigkeit der Reaktion fragwürdig ist oder eine Reaktion gar nicht erfolgen kann, weil der Patient hierzu behindert, unfähig oder unwillig ist.

Gemäss Anspruch 6 kann deshalb die Reaktion des Patienten auf den am gleichen Testort veränderlichen Wert des Stimulus durch eine Messung der Reaktion der Pupille des Patienten festgestellt werden. Gemäss den Ansprüchen 8 und 9 kann die Reaktion des Patienten auf den am gleichen Testort veränderlichen Wert des Stimulus auch durch eine Messung von Potentialen an einem Körperorgan festgestellt werden, indem beispielsweise die Hirnpotentiale des Patienten gemessen werden.

Ausführungsbeispiele des erfindungsgemässen Verfahrens werden nachstehend an der Zeichnungen erläutert. Es zeigen:
Fig. 1 ein Prinzipschema einer Einrichtung zur Durchführung des Verfahrens, und
Fig. 2 eine zur Durchführung des Verfahrens modifizierte Vorrichtung mit einem Perimeter.

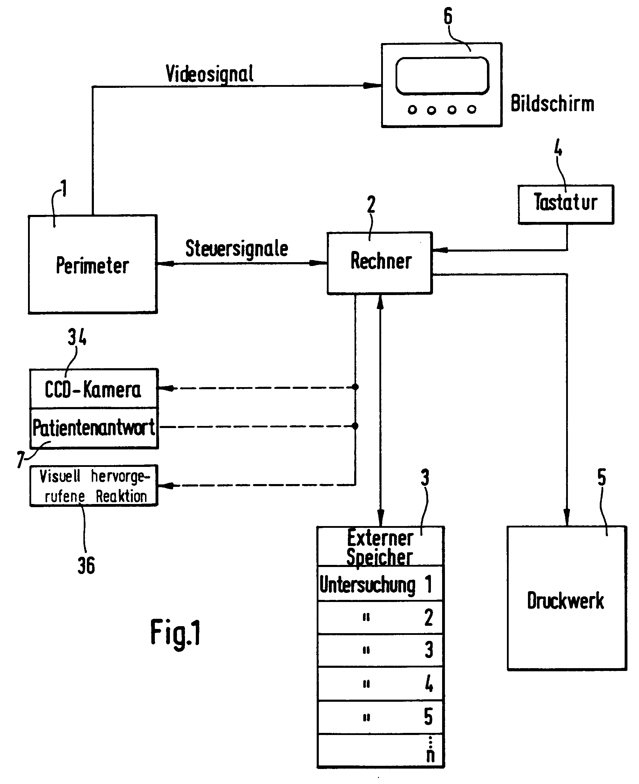

Im Prinzipschema der Fig. 1 ist ein Perimeter 1 dargestellt, das zur wechselseitigen Uebertragung von Steuersignalen mit einem Rechner 2 mit internem Speicher verbunden ist und das an einer bestimmten Beobachterstelle das zu untersuchende Patientenauge in einer Beobachtungsrichtung fixiert. Hierbei werden dem Auge mit geeigneten Mitteln Stimuli zunehmenden Wertes im Umfeld der Beobachtungsachse dargeboten. Dem Rechner 2 ist wahlweise ein externer Speicher 3 zugeordnet. Im internen Speicher des Rechners 2 und/oder im externen Speicher 3 werden von den verschiedenen Untersuchungen die ermittelten Schwellenwerte der lokalen Unterschiedsempfindlichkeit bezüglich der Stimuli abgespeichert. Um den Rechner 2 Befehle zuzuleiten, ist eine Tastatur 4 vorhanden.

Mittels eines vom Rechner 2 gesteuerten Druckwerks 5 können die im internen oder externen Speicher 3 gespeicherten Untersuchungsresultate in numerischer oder graphischer Form zur Anzeige gebracht werden. Ueber einen Monitor 6 können das Patientenauge und seine Ausrichtung beobachtet werden bzw. kann die Kommunikation mit dem Rechner 2 bewerkstelligt werden.

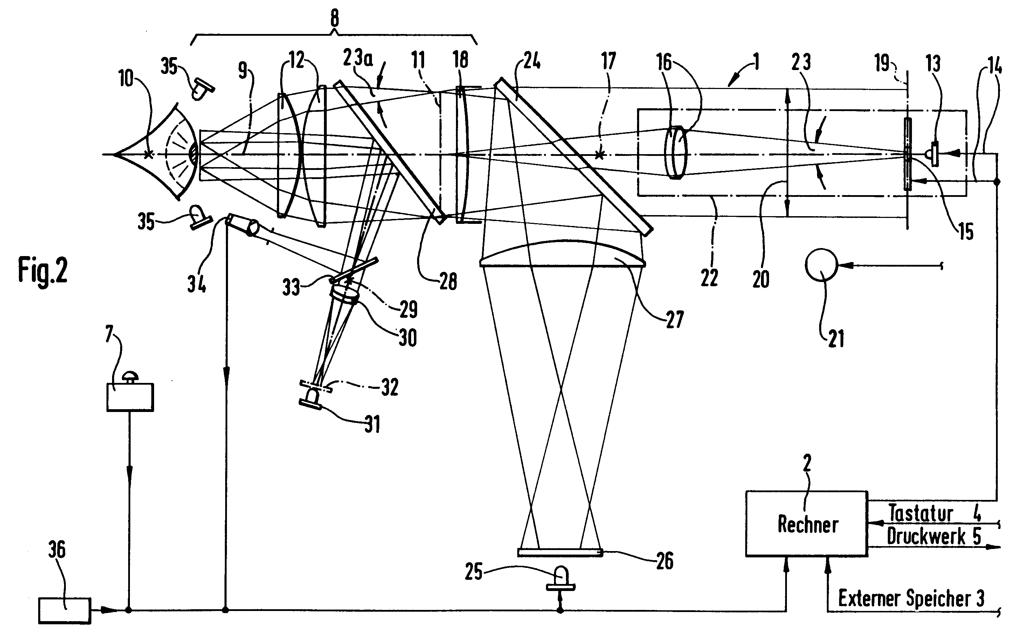

Wie bereits erwähnt, erzeugt die Kombination des Perimeters 1 und des Rechners 2 für jeden durch diese bestimmten Testort einen Stimulus, dessen Wert am gleichen Testort einen kontinuierlichen Stimulus, dessen Wert am gleichen Testort gegebenenfalls eine getaktete Konfiguration mit zunehmendem Wert hat. Man kann diesen Stimulus deshalb als Rampenstimulus bezeichnen. Dieser Rampenstimulus fängt mit einem leicht unterschwelligen, alterskorrigierten Normalwert an und präsentiert sich dem Patientenauge mit definiert zunehmender Lichtmenge, bis der Stimulus vom Auge wahrgenommen wird. Die Lichtmenge kann hierbei, wie anhand der Fig. 2 noch gezeigt wird, entweder als veränderliche, ansteigende Lichtintensität oder als veränderliche, zunehmende geometrische Grösse des Stimulus gesteuert sein.

Wahrnehmungssignale können auf verschiedene Arten erzeugt werden, wie dies in Fig. 1 durch entsprechende Kästchen und gestrichelte Verbindungslinien mit dem Perimeter 1 und dem Rechner 2 angedeutet ist.

Erstens kann die Wahrnehmung des Stimulus in an sich bekannter Weise dem Rechner 2 durch den Patienten selbst signalisiert werden, indem dieser bei der subjektiven Wahrnehmung einen an den Rechner 2 angeschlossenen Antwortknopf 7 (vgl. auch Fig. 2) drückt.

Zweitens kann die Wahrnehmung des Stimulus entweder allein oder zusammen mit der Antwortbetätigung durch den Patienten dadurch signalisiert werden, dass in objektiver Weise die Veränderung der Pupille des Patientenauges bei der subjektiven Wahrnehmung des Stimulus gemessen wird, beispielsweise mittels einer auf die Pupille gerichteten Infrarot-CCD-Kamera 34 (vgl. auch Fig. 2), und das entsprechende Signal dem Rechner 2 zugeleitet wird.

Drittens ist es auch möglich, bei der Wahrnehmung des Stimulus auftretende Potentiale eines Körperorgans des Patienten, beispielsweise Hirnpotentiale, mittels einer geeigneten Vorrichtung 36 (vgl. auch Fig. 2) zu messen und das entsprechende Signal dem Rechner 2 zuzuleiten.

Das vorliegende Verfahren weist die folgenden Vorteile auf:
a) Die Untersuchung kann um einen Faktor bis zu fünf schneller durchgeführt werden als bei den bekannten Verfahren.
b) Bei Anwendung einer Messung der Reaktion der Pupille oder einer Messung der Hirnpotentiale liegt eine objektive Messung vor, so dass die Untersuchung vom Patienten unbeeinflussbar ablaufen wird und eine Simulation durch den Patienten ausgeschlossen ist.
c) Die Untersuchung kann mit behinderten, jugendlichen, senilen oder andersweitig "unfähigen" Patienten durchgeführt werden.
d) Die Zuverlässigkeit der Untersuchung und damit die Genauigkeit der Untersuchung nehmen zu.

Anhand der Fig. 2 wird nun eine praktische Ausführungsform des eigentlichen Perimeters 1 beschrieben. Diese Ausführungsform stimmt in weiten Teilen mit der in der Europäischen Patentanmeldung 0 363 610 dargestellten und beschriebenen Ausführungsform eines Perimeters überein, wobei jedoch grundsätzliche Abweichungen von diesem Stand der Technik aufgrund der Erfordernisse zur Durchführung des erfindungsgemässen Verfahrens vorliegen.

Gemäss Fig. 2 weist das eigentliche Perimeter 1 ein Okular 8 auf, dessen optische Achse 9 mit der Beobachterachse eines an der Beobachterstelle 10 für eine Untersuchung fixierten Beobachterauges zusammenfällt. Die Linsen 12 des Okulars 8 sind auf eine Zwischenbildebene 11 (diese kann in Wirklichkeit leicht gekrümmt sein) fokussiert und derart geschliffen, dass sie die von einem Punkt der Zwischenbildebene 11 ausgehenden, divergierenden Strahlen parallel richten und gegen die Beobachterstelle 10 umlenken. Mit Bezug auf die Zwischenbildebene 11 bildet das Okular 8 eine Lupe. Mit dem parallelen Ausrichten der Strahlen durch die Linsen 12 gegen die Beobachterstelle 10 wird die Möglichkeit geschaffen, dass sich während einer Untersuchung das Beobachterauge in bestimmten Grenzen in einer Ebene senkrecht zur optischen Achse 9 verschieben darf, ohne dass deswegen ein dargebotener Stimulus unscharf (oder überhaupt nicht) wahrgenommen wird, oder dessen geometrische Lage vom Patienten verfälscht erkannt wird.

Weiter weist das eigentliche Perimeter 1 eine Lichtquelle 13 vorzugsweise in Form einer Leuchtdiode auf, deren Lichtstärke vom Rechner 3 über eine Leitung 14 steuerbar ist. Die Verwendung einer Leuchtdiode hat den Vorteil, dass die Lichtquelle 13 verzögerungsfrei steuerbar ist. Zudem kann sie zur Durchführung der Farbperimetrie leicht gegen eine farbige Leuchtdiode ausgetauscht werden. Es können indessen auch Halogenlampen oder Laserröhren als Lichtquelle verwendet werden.

Vom Licht der Lichtquelle 13 wird mittels einer Blende 15, welche die Form und die Grösse eines Stimulus bestimmt, ein gebündelter Lichtstrahl ausgeblendet. Auch die Blende 15 bzw. deren Oeffnung ist über eine weitere Leitung 14 vom Rechner 3 steuerbar. Die Steuerbarkeit sowohl der Lichtquelle 13 als auch der Blende 15 entspricht dem Merkmal des vorliegenden Verfahrens, dass der kontinuierlich gegebenenfalls taktweise zunehmende Wert des Stimulus die Lichtintensität oder die Grösse des Stimulus sein kann.

Auf die Blende 15 ist ein Objektiv bestehend aus den Linsen 16 gerichtet, das von der Blendenöffnung bzw. einem Stimulus auf der Zwischenbildebene 11 ein reelles Zwischenbild erzeugt. Die Linsen 16 sind mit ihrer optischen Achse 17 Parallel zur Achse 9 des Okulars 8 orientiert. Das auf der Zwischenbildebene 11 erzeugte reelle Zwischenbild des Stimulus wird vom Beobachterauge an der Beobachterstelle 10 durch das Okular 8, zu welchem auch eine Feldlinse 18 gehört, vergrössert wahrgenommen.

Die Lichtquelle 13 und die Blende 15 sind gemeinsam in einer Ebene senkrecht zur optischen Achse 17 der Linsen 16 verschiebbar. In der Ebene 19 der Blende 15 können daher die Stimuli am vom Rechner 2 frei wählbaren, koordinatenmässig bestimmten Orten im vom den Linsen 16 erfassten Umfeld dargeboten werden. Ein vom Rechner 2 gesteuerter Antrieb 21 dient der Lageverschiebung der Lichtquelle 13, der Blende 15 und der Linsen 16, welche eine bauliche Einheit 22 bilden und gemeinsam in einer Ebene senkrecht zur optischen Achse 17 verschiebbar sind. Vom Antrieb 21 wird jeweils die ganze Einheit 22 an den vom Rechner 2 befohlenen Ort bewegt. Durch die Beweglichkeit der Linsen 16 ist deren Durchmesser und damit die Grösse des Winkels 23 bei kleinem Abstand zur Blende 15 wählbar. Der Winkel 23 ist, mindestens in grober Näherung, vorzugsweise gleich dem Winkel 23a, dessen Grösse den Abstand von zwei von der Zwischenbildebene 11 ausgehenden, divergierenden und von der Doppellinse 12 parallel gerichteten Strahlen. Der Winkel 23 beträgt bei diesem Beispiel max. 20^{o}; seine Grösse bestimmt die Abweichungstoleranz des Patientenauges von der Beobachterstelle 10.

Um für einen Patienten das ganze beobachtbare Umfeld der Beobachterachse 9 gleichmässig auszuleuchten, ist in der optischen Achse 17 ein gegen die Zwischenbildebene 11 gerichteter, teilweise lichtdurchlässiger Spiegel 24 angeordnet, der einerseits das durch die Linsen 16 durchtretende Licht durchlässt und andererseits das von einer Lichtquelle 25 erzeugte Licht gegen die Zwischenbildebene 11 umlenkt. Für eine gleichmässige Ausleuchtung des Umfeldes der Beobachterachse 9 sind zwischen der Lichtquelle 25 und dem Umlenkspiegel 24 eine Diffusorscheibe 26 und eine zum Beispiel als Fresnellinse gestaltete Sammellinse 27 angeordnet. Die Diffusorscheibe 26 kann für die Durchführung der Farbperimetrie zusätzlich als Farbfilter gestaltet sein.

Um einem Beobachterauge die Orientierung mit Bezug auf die Beobachterachse zu erleichtern, wird dem Patienten auf dieser Achse eine hell leuchtende Marke (Fixationsmarke) als Orientierungshilfe angeboten. Diesem Zweck dient eine zwischen der Zwischenbildebene 11 und der Doppellinse 12 angeordneter, teilweise durchlässiger, gegen die Beobachterstelle 10 gerichteter Umlenkspiegel 28. Den Schnittpunkt zwischen dem Umlenkspiegel 28 und der Beobachterachse 9 schneidet weiter die optische Achse 29 von zwei Linsen 30, die axial mit einer Lichtquelle 31 und einer Blende 32 ausgerichtet sind. Die Blende 32 bestimmt die Form und die Grösse der Fixationsmarke.

Ueber einen weiteren, in der optischen Achse 29 angeordneten Umlenkspiegel 33, der durchlässig für sichtbares Licht und reflektierend für infrarotes Licht ist, ist eine infrarot-empfindliche CCD-Kamera 34 auf die Beobachterachse 9 geschaltet, mit der ein Patientenauge während einer Untersuchung beobachtet werden kann. Zu diesem Zweck wird das Patientenauge an der Beobachterstelle 10 mit Infrarotlicht von Leuchtdioden 35 unsichtbar beleuchtet. Somit liefert die CCD-Kamera 34 ein dem Rechner 2 zugeführtes Signal eines Bildes der Pupille des Patientenauges, so dass der Rechner 2 die bei der Wahrnehmung eines Stimulus bestimmten Wertes durch das Patientenauge dann auftretende Pupillenveränderung ohne aktives Eingreifen des Patienten feststellen kann.

Schliesslich ist noch eine zur Erfassung visuell erregter Reaktionen eines Körperorgans des Patienten, beispielsweise von Hirnpotentialen, vorgesehene Vorrichtung 36 schematisch dargestellt, welche bei ihrer Verwendung ebenfalls an den Rechner angeschlossen wird und dazu dient, die Wahrnehmung eines Stimulus durch das Patientenauge ohne aktives Eingreifen des Patienten zu signalisieren.

Die Linsen 16 und die Feldlinse 18 können entfallen, wenn die Lichtquelle 13 und die Blende 15 soweit gegen das Okular 8 verschoben werden, dass die Ebene 19 mit der Zwischenbildebene 11 zusammenfällt (nicht dargestellt). Dies könnte beispielsweise mit einem Bildschirm in der Ebene 11 realisiert werden (nicht dargestellt). Wird in der Zwischenbildebene 11 ein Bildschirm zur Darbietung des Stimulus angeordnet, besteht die Möglichkeit, den Stimulus dunkler als das Umfeld zu wählen.

Bei beiden Ausführungsbeispielen wird ein Abstand der Beobachterstelle 10 von der Zwischenbildebene 11 gewählt, der kleiner ist als die Sehweite für deutliches Sehen.

## Patentansprüche

1. Verfahren zum Bestimmen des Schwellenwertes der Wahrnehmung von Licht an wählbaren Testorten des Gesichtsfeldes eines Auges mit Hilfe eines von einem Rechner gesteuerten Perimeters, wobei an den durch den Rechner wählbaren Testorten im Umfeld einer Beobachtungsachse mittels einer Lichtquelle Stimuli unterschiedlicher Werte dargeboten werden, bis aufgrund von dem Rechner zugeführten Patientenreaktionen auf die Stimuli ein Schwellenwert der Wahrnehmung ermittelt ist, dadurch gekennzeichnet, dass dem Patienten an jedem Testort jeweils nur einmal ein Stimulus kontinuierlich zunehmenden Wertes bis zum Auftreten einer Patientenreaktion dargeboten wird, und dass der Wert des Stimulus beim Auftreten der Patientenreaktion als charakteristische Grösse für den Schwellenwert der Wahrnehmung festgehalten wird.

2. Verfahren nach Anspruch 1, dadurch gekennzeichnet, dass an jedem Testort ein getakteter Stimulus zunehmenden Wertes dargeboten wird.

3. Verfahren nach einem der Ansprüche 1 oder 2, dadurch gekennzeichnet, dass der zunehmende Wert des Stimulus die Lichtintensität des Stimulus ist.

4. Verfahren nach einem der Ansprüche 1 oder 2, dadurch gekennzeichnet, dass der zunehmende Wert des Stimulus die Stimulusgrösse ist.

5. Verfahren nach einem der Ansprüche 1 bis 4, dadurch gekennzeichnet, dass die Reaktion des Patienten auf den am gleichen Testort veränderlichen Wert des Stimulus mittels der Betätigung eines Antwortknopfes durch den Patienten festgestellt wird.

6. Verfahren nach einem der Ansprüche 1 bis 4, dadurch gekennzeichnet, dass die Reaktion des Patienten auf den am gleichen Testort veränderlichen Wert des Stimulus durch eine Messung der Reaktion der Pupille des Patienten festgestellt wird.

7. Verfahren nach Anspruch 6, dadurch gekennzeichnet, dass zur Messung der Reaktion der Pupille des Patienten eine Infrarot-CCD-Kamera verwendet wird.

8. Verfahren nach einem der Ansprüche 1 bis 4, dadurch gekennzeichnet, dass die Reaktion des Patienten auf den am gleichen Testort veränderlichen Wert des Stimulus durch eine Messung von Potentialen an einem Körperorgan des Patienten festgestellt wird.

9. Verfahren nach Anspruch 8, dadurch gekennzeichnet, dass die Hirnpotentiale des Patienten gemessen werden.

10. Einrichtung zur Untersuchung des Gesichtsfeldes des Auges eines Patienten, mit einer Lichtquelle (13), einer dieser nachgeschalteten Blende (15) und einem optischen System (8, 16), durch welches die Lichtstrahlen der Lichtquelle (13) auf die Netzhaut des auf einer Beobachtungsachse (9) an einer Beobachtungsstelle (10) befindlichen Auges ausrichtbar sind, sowie mit einem Rechner (2) zur Erzeugung von Stimuli unterschiedlicher Werte mittels der Lichtquelle (13) an wählbaren Testorten im Umfeld der Beobachtungsachse (9) und mit an den Rechner (2) angeschlossenen Mitteln (7, 36, 37) zur Erzeugung einer Patientenreaktion auf die Stimuli zur Ermittlung eines Schwellenwertes ihrer Wahrnehmung durch den Patienten, dadurch gekennzeichnet, dass der Rechner (2) so ausgebildet ist, dass an jedem Testort jeweils nur einmal ein Stimulus kontinuierlich zunehmenden Wertes bis zum Auftreten der Patientenreaktion dargehoten wird.

11. Einrichtung nach Anspruch 10, dadurch gekennzeichnet, dass der Rechner (2) zur Steuerung einer taktweise zunehmenden Lichtintensität der Lichtquelle (13) ausgebildet ist.

12. Einrichtung nach Anspruch 10, dadurch gekennzeichnet, dass die Oeffnung der Blende (14) verstellbar ist, und dass der Rechner (2) zur Steuerung einer taktweise zunehmenden Oeffnung der Blende (14) ausgebildet ist.

## Claims

1. Process for determining the threshold value of visibility of light at selectable test points at the visual field of an eye with the aid of a computer-controlled perimeter, and that stimuli of different values are offered by a light source at computer selectable test points in the area around an observation axis until a threshold value of visibility is determined based on patient reactions to said stimuli as delivered by the computer, **characterized in that** the patient is offered only once at each test point a continuously increasing stimulus until a patient reaction is offered, and that the stimulus value on occurrence of the patient reaction is taken as a characteristic value for the threshold value of visibility.

2. Process according to Claim 1, **characterized in that** a pulsed stimulus of increasing value is offered at each test point.

3. Process according to one of Claims 1 or 2, **characterized in that** the increasing value of the stimulus is the light intensity of the stimulus.

4. Process according to one of Claims 1 or 2, **characterized in that** the increasing value of the stimulus is the size of the stimulus.

5. Process according to one of Claims 1 to 4, **characterized in that** the reaction of a patient to a stimulus value which is changeable at the same test point is determined by means of a response button to be operated by the patient.

6. Process according to one of Claims 1 to 4, **characterized in that** the reaction of a patient to a stimulus value which is changeable at the same test point is determined by measuring the reaction of the patient's pupil.

7. Process according to Claim 6, **characterized in that** an infrared CCD-camera is used for measuring the reaction of a patient's pupil.

8. Process according to one of Claims 1 to 4, **characterized in that** the reaction of a patient to a stimulus value which is changeable at the same test point is determined by measuring potentials on a body organ of the patient.

9. Process according to Claim 8, **characterized in that** the brain potentials of a patient are measured.

10. Apparatus for examining the visual field of the eye of a patient, comprising a light source (13), downstream thereof a diaphragm (15) and an optical system (8, 16) by means of which light rays of the light source (13) can be aligned with the retina of an eye located on an observation axis (9) at an observation point (10), and a computer (2) for generating stimuli of different values by means of the light source (13) at selectable test points in the area around the observation axis (9), and with computer-connected (2) means (7, 36, 37) for generating a patient reaction to the stimuli for the purpose of determining a threshold value of their awareness by a patient, **characterized in that** the computer (2) is designed in such a manner that at each test point a respective stimulus of continuously increasing value is offered only once until a patient reaction occurs.

11. Apparatus according to Claim 10, **characterized in that** the computer (2) is designed for controlling an increasing pulsed light intensity of the light source (13).

12. Apparatus according to Claim 10, **characterized in that** the diaphragm aperture (14) is adjustable, and that the computer is designed for controlling a diaphragm aperture (14) of pulsed increase.

## Revendications

1. Procédé pour déterminer la valeur seuil de la perception de lumière en des endroits de contrôle susceptibles d'être choisis du champ visuel d'un oeil, à l'aide d'un périmètre commandé par un calculateur, tandis qu'aux endroits de contrôle susceptibles d'être choisis par le calculateur, dans le champ périphérique d'un axe d'observation, des stimuli de différentes valeurs sont présentés au moyen d'une source de lumière jusqu'à ce que, sur la base des réactions des patients aux stimuli amenées au calculateur, une valeur seuil de la perception soit déterminée,
procédé caractérisé en ce qu'au patient, à chaque endroit de contrôle, est présenté respectivement une seule fois un stimulus d'une valeur croissant de façon continue jusqu'à l'apparition d'une réaction du patient, et en ce que la valeur du stimulus lors de l'apparition de la réaction du patient est retenue en tant que grandeur caractéristique pour la valeur seuil de la perception.

2. Procédé selon la revendication 1, caractérisé en ce qu'en chaque endroit de contrôle est présenté un stimulus synchronisé de valeur croissante.

3. Procédé selon une des revendications 1 ou 2, caractérisé en ce que la valeur croissante du stimulus est l'intensité lumineuse du stimulus.

4. Procédé selon une des revendications 1 ou 2, caractérisé en ce que la valeur croissante du stimulus est la grandeur du stimulus.

5. Procédé selon une des revendications 1 à 4, caractérisé en ce que la réaction du patient à la valeur variable du stimulus au même endroit de contrôle est constatée au moyen de l'actionnement d'un bouton de réponse par le patient.

6. Procédé selon une des revendications 1 à 4, caractérisé en ce que la réaction du patient à la valeur variable du stimulus au même endroit de contrôle est constatée grâce à une mesure de la réaction de la pupille du patient.

7. Procédé selon la revendication 6, caractérisé en ce que, pour la mesure de la réaction de la pupille du patient, une camera infrarouge à couplage de charge est utilisée.

8. Procédé selon une des revendications 1 à 4, caractérisé en ce que la réaction du patient à la valeur variable du stimulus au même endroit de contrôle est constatée grâce à une mesure de potentiels sur un organe du corps du patient.

9. Procédé selon la revendication 8, caractérisé en ce que les potentiels du cerveau du patient sont mesurés.

10. Dispositif pour la détermination du champ visuel de l'oeil d'un patient, avec une source de lumière (13), un diaphragme (15) branché à la suite de celle-ci et un système optique (8,16), grâce auquel les rayons lumineux de la source de lumière (13) sont susceptibles d'être orientés sur la rétine de l'oeil se trouvant à un emplacement d'observation (10) sur un axe d'observation (9), ainsi qu'avec un calculateur (2) pour obtenir des stimuli de valeurs différentes, au moyen de la source de lumière (13), à des endroits de contrôle susceptibles d'être choisis dans le champ périphérique de l'axe d'observation (9), et avec des moyens (7, 36, 37) raccordés au calculateur (2) pour obtenir une réaction aux stimuli, pour la détermination d'une valeur seuil de leur perception par le patient,
procédé caractérisé en ce que le calculateur (2) est réalisé de façon qu'à chaque endroit de contrôle respectivement seulement une fois un stimulus de valeur continuellement croissante est présenté jusqu'à l'apparition de la réaction du patient.

11. Dispositif selon la revendication 10, caractérisé en ce que le calculateur (2) est prévu pour la commande d'une intensité lumineuse de la source de lumière croissant de façon cadencée.

12. Dispositif selon la revendication 10, caractérisé en ce que l'ouverture du diaphragme (14) est réglable, et en ce que le calculateur (2) est réalisé pour la commande d'une ouverture du diaphragme (14) croissant de façon cadencée.
